# EUROPÄISCHE PATENTSCHRIFT

(11) **EP 0 920 337 B1**
(45) Veröffentlichungstag und Bekanntmachung des Hinweises auf die Patenterteilung: **16.04.2003**
(21) Anmeldenummer: 96904845.3
(22) Anmeldetag: 22.02.1996
(51) Int. Cl.: A61K 47/02, A61K 9/107, A61K 9/52, A61K 9/48, A61K 9/22, A61K 9/20, A61K 9/14, A61K 9/02

(54) **VERWENDUNG VON ANORGANISCHEN AEROGELEN IN DER PHARMAZIE**
USE OF INORGANIC AEROGELS IN PHARMACY
UTILISATION D'AEROGELS INORGANIQUES EN PHARMACIE

(30) Priorität: 22.02.1995 DE 19506141
(43) Veröffentlichungstag der Anmeldung: 09.06.1999
(73) Patentinhaber: CABOT CORPORATION, Boston, Massachusetts 02210-2019 (US)
(72) Erfinder: SCHWERTFEGER, Fritz, D-60529 Frankfurt am Main (DE); ZIMMERMANN, Andreas, D-64347 Griesheim (DE); KREMPEL, Harald, D-64342 Seeheim-Jugenheim (DE)
(74) Vertreter: Luderschmidt, Schüler & Partner GbR
(86) Internationale Anmeldenummer: EP9600731
(87) Internationale Veröffentlichungsnummer: WO96025950

(56) Entgegenhaltungen:
- EP-A- 0 163 178
- EP-A- 0 255 000
- WO-A-95/01165
- DE-A- 2 652 163
- US-A- 4 123 511
- US-A- 4 185 088
- US-A- 4 411 885
- DATABASE WPI Section Ch, Week 9411 Derwent Publications Ltd., London, GB; Class B07, AN 94-089171 XP002006862 & JP,A,06 040 714 (SHIONOGI & CO LTD) , 15.Februar 1994

## Beschreibung

Die Erfindung betrifft die Verwendung von durch Silylierung oberflächen-modifizierten anorganischen Aerogelen als Hilfsstoff und/oder Trägermaterial für pharmazeutische Wirkstoffe und/oder Zubereitungen.

Aerogele, insbesondere solche mit Porositäten über 60 % und Dichten unter 0,6 g/cm³, weisen eine äußerst geringe thermische Leitfähigkeit auf und finden deshalb Anwendung als Wärmeisolationsmaterial wie z.B. in der EP-A-0 171 722 beschrieben. Darüber hinaus ist die Verwendung von Aerogelen für Cerenkov-Detektoren aufgrund ihrer für Feststoffe sehr geringen Brechzahl bekannt. Weiterhin ist in der Literatur aufgrund der besonderen akustischen Impedanz der Aerogele eine mögliche Verwendung als Impedanzanpassung z.B. im Ultraschallbereich beschrieben.

Aerogele im weiteren Sinn, d.h. im Sinne von "Gel mit Luft als Dispersionsmittel", werden durch Trocknung eines geeigneten Gels hergestellt. Unter den Begriff "Aerogel" in diesem Sinne, fallen Aerogele im engeren Sinn, Xerogele und Kryogele. Dabei wird ein getrocknetes Gel als Aerogel im engeren Sinn bezeichnet, wenn die Flüssigkeit des Gels bei Temperaturen oberhalb der kritischen Temperatur und ausgehend von Drücken oberhalb des kritischen Druckes weitestgehend entfernt wird. Wird die Flüssigkeit des Gels dagegen unterkritisch, beispielsweise unter Bildung einer Flüssig-Dampf-Grenzphase entfernt, dann bezeichnet man das entstandene Gel als Xerogel.

Bei der Verwendung des Begriffs Aerogele in der vorliegenden Anmeldung handelt es sich um Aerogele im weiteren Sinn, d.h. im Sinn von "Gel mit Luft als Dispersionsmittel".

Darüber hinaus kann man die Aerogele grundsätzlich in anorganische und organische Aerogele unterteilen.
Anorganische Aerogele sind schon seit 1931 bekannt (S.S. Kistler, Nature 1931, 127, 741). Seitdem sind aus unterschiedlichsten Ausgangsmaterialien Aerogele dargestellt worden. Dabei konnten z.B. SiO₂-, Al₂O₃-, TiO₂-, ZrO₂-, SnO₂-, Li₂O-, CeO₂-, V₂O₅-Aerogele, und Mischungen aus diesen hergestellt werden (H.D. Gesser, P.C. Goswami, Chem. Rev. 1989, 89, 756ff).
Seit einigen Jahren sind auch organische Aerogele aus unterschiedlichsten Ausgangsmaterialien, wie z.B. aus Melaminformaldehyd, bekannt (R.W. Pekala, J. Mater, Sci. 1989, 24, 3221).

Anorganische Aerogele können dabei auf unterschiedlichsten Wegen hergestellt werden.

Beispielsweise können SiO₂-Aerogele durch saure Hydrolyse und Kondensation von Tetraethylorthosilikat in Ethanol hergestellt werden. Dabei entsteht ein Gel, das durch überkritische Trocknung unter Erhaltung der Struktur getrocknet werden kann. Auf dieser Trocknungstechnik basierende Herstellungsverfahren sind z.B. aus der EP-A-0 396 076 oder der WO 92/03378 bekannt.

Eine Alternative bietet ein Verfahren zur unterkritischen Trocknung von SiO₂-Gelen, wenn diese vor der Trocknung mit einem chlorhaltigen Silylierungsmittel umgesetzt werden. Das SiO₂-Gel kann dabei beispielsweise durch saure Hydrolyse von Tetraalkoxysilanen in einem geeigneten organischen Lösungsmittel mittels Wasser erhalten werden. Nach Austausch des Lösungsmittels gegen ein geeignetes organisches Lösungsmittel wird in einem weiteren Schritt das erhaltene Gel mit einem Silylierungsmittel umgesetzt. Das dabei entstehende SiO₂-Gel kann anschließend aus einem organischen Lösungsmittel heraus an der Luft getrocknet werden. Damit können Aerogele mit Dichten unter 0,4 g/cm³ und Porositäten über 60 % erreicht werden.

Das auf dieser Trocknungstechnik basierende Herstellungsverfahren ist ausführlich in der WO 94/25149 beschrieben.

Die oben beschriebenen Gele können darüber hinaus vor der Trocknung in der alkohol-wäßrigen Lösung mit Tetraalkoxysilanen versetzt und gealtert werden, um die Geinetzwerkstärke zu erhöhen, z.B. wie in der WO 92/20623 offenbart.

Ferner kann das SiO₂-Gel auch auf Basis von Wasserglas hergestellt werden. Das auf dieser Technik basierende Herstellungsverfahren ist aus der DE-A-43 42 548 bekannt.

In der deutschen Patentanmeldung 19502453.2 wird darüber hinaus die Verwendung von chlorfreien Silylierungsmitteln beschrieben.

Die durch überkritische Trocknung erhaltenen Aerogele sind, je nach dem speziell angewendeten Verfahren hydrophil oder kurzfristig hydrophob. Langfristig sind sie jedoch hydrophil.

Dies kann durch einen Hydrophobisierungsschritt während der überkritischen Trocknung vermieden werden. Ein solches Verfahren ist aus der EP-A-0 396 076 bekannt.

Unterkritisch getrocknete Aerogele sind bedingt durch ihr Herstellungsverfahren (Silylierung vor der Trocknung) dauerhaft hydrophob.

Die Verwendung von kolloidalem Silica in therapeutischen Kupfer-Zusammensetzungen ist beispielsweise aus der US-A-4,123,511 bekannt.

Ebenfalls bekannt ist die Verwendung von organischen Aerogelen in der Medizin (WO 95/01165).

In der DE-A 26 52 163 wird ein Verfahren zur Verminderung der für eine gewisse biologische Wirkung erforderlichen Menge biologisch aktiver Substanz offenbart, wobei die biologisch aktive Substanz oder ein Vorläuferstoff für diese, nebst eventuellen Zusätzen, auf einem inaktiven, festen, feinverteilten, anorganischen Träger gleichmäßig verteilt wird. Offenbart werden dabei als bevorzugte Träger Silziumdioxid, gefälltes Siliziumdioxid oder pyrogenes Siliziumdioxid. Diese Siliziumdioxide können hinsichtlich Säuregrad, Alkalinität sowie Hydrophilie oder Hydrophobie modifiziert werden.

In der JP-A-06/040 714 (Derwent, AN 94-089171) wird die Verwendung eines porösen Silicas, welches eine hohe Ölabsorption aufweist, als Trägermaterial für Medikamente und Herbizide offenbart. Die Herstellung des porösen Silicas erfolgt dabei durch überkritische Trocknung eines Silica-Hydrogels, welches durch Umsetzung von alkalischen Metallsilikaten mit Mineralsäuren erhalten wird.

Im Research Disclosure Nr. 35267, Seite 562 (1993) wird die Verwendung von Aerogelen und Kryogelen, die gegebenenfalls modifiziert sein können, in kosmetischen Produkten beschrieben.

Aufgabe der vorliegenden Erfindung war es, nach neuen Anwendungen für Aerogele zu suchen.

Es wurde nun überraschend gefunden, daß anorganische Aerogele als Hilfsstoff und/oder Trägermaterial für pharmazeutische Wirkstoffe und/oder Zubereitungen geeignet sind.

Unter einem anorganischen Aerogel ist in der vorliegenden Anmeldung ein Aerogel zu verstehen, das auf der Basis von anorganischen Materialien hergestellt wurde.

Unter den Begriff "Aerogele auf der Basis von anorganischen Materialien" fallen insbesondere solche Aerogele die durch Silylierung modifiziert wurden.

Bevorzugt sind Aerogele überwiegend aus SiO₂, Al₂O₃, TiO₂, ZrO₂ oder Mischungen davon. Diese können, je nach Verwendung, hydrophile und/oder hydrophobe Oberflächengruppen (z.B. OH, OR, R) aufweisen. Die Herstellung von Aerogelen mit hydrophilen und/oder hydrophoben Oberflächengruppen kann dabei nach allen dem Fachmann bekannten Verfahren durchgeführt werden. Besonders bevorzugt sind hydrophile oder hydrophobe SiO₂-haltige Aerogele, insbesondere SiO₂-Aerogele.

Darüber hinaus wurde überraschend gefunden, daß durch die Wahl eines geeigneten hydrophilen oder hydrophoben Aerogels entsprechende Stotfe, mit denen das Aerogel beladen wurde, beschleunigt bzw. verzögert freigesetzt werden können. Weiterhin können Aerogele als Dispergierungsmittel für Dispersionen von festen, flüssigen oder gasförmigen Stoffen in feste oder flüssige Medien eingesetzt werden. Darüber hinaus können mit hydrophilen und/oder hydrophoben Stoffen beladene hydrophile oder hydrophobe Aerogele problemlos in hydrophile und/oder hydrophobe, flüssige, halbfeste bzw. feste Medien eingearbeitet werden, insbesondere, um mit Hilfe von hydrophilen Aerogelen hydrophobe (d.h. lipophile) Stoffe in flüssige und/oder halbfeste hydrophile Dispersionsmedien und mit Hilfe von hydrophoben Aerogelen hydrophile Stoffe in flüssige, hydrophobe Disperionsmedien einzutragen. Hydrophobe Aerogele beispielsweise, schwimmen auf hydrophilen, wäßrigen Medien auf, wodurch magensaftflotierende Arzneistoff-Trägersysteme möglich sind. Ferner können auch flüssige, hydrophile oder hydrophobe Stoffe in feste, frei rieselfähige Pulver oder Granulate überführt werden. Damit ist eine problemlose Verarbeitung z.B. zu Tabletten, Kapseln oder Zäpfchen möglich. Weiterhin ist mit entsprechenden Aerogelen auch die Herstellung von Lotionen, Cremes und Gelen mit und ohne Peeling-Effekt möglich. Stoffe im Sinne dieser Anwendungen sind in der Pharmazie verwendbare Stoffe, z.B. Arzneistoffe, Duftstoffe und Geschmackstoffe.

Die Erfindung wird im folgenden anhand von Ausführungsbeispielen näher beschrieben, ohne dadurch beschränkt zu werden.

### Herstellungsbeispiele

### Beispiel 1

### Darstellung eines dauerhaft hydrophoben Aerogels

1 I einer Natriumwasserglaslösung (mit einem Gehalt von 7 Gew.-% SiO₂ und einem Na₂O:SiO₂ Verhältnis von 1:3,3) wurde zusammen mit 0,5 I eines sauren lonenaustauscherharzes (Styroldivinylbenzolcopolymer mit Sulfonsäuregruppen, handelsüblich unter dem Namen ®Duolite C20) gerührt, bis der pH-Wert der wäßrigen Lösung 2,3 war. Anschließend wurde das lonenaustauscherharz abfiltriert und die wäßrige Lösung mit 1 molarer NaOH-Lösung auf einen pH-Wert von 5,0 eingestellt. Danach wurde das entstandene Gel noch 3 Stunden bei 85°C gealtert und anschließend das Wasser mit 3 I Aceton gegen Aceton ausgetauscht. Anschließend wurde das acetonhaltige Gel mit Trimethylchlorsilan silyliert (5 Gew.-% Trimethylchlorsilan pro Gramm nasses Gel). Die Trocknung des Gels erfolgte an Luft (3 Stunden bei 40°C, dann 2 Stunden bei 50°C und 12 Stunden bei 150°C).

Das so erhaltene, transparente Aerogel hatte eine Dichte von 0,15 g/cm³, seine spezifische Oberfläche nach BET lag bei 480 m²/g und es war dauerhaft hydrophob.

### Beispiel 2

### Darstellung eines hydrophilen Aerogels

Das in Beispiel 1 hergestellte, dauerhaft hydrophobe Aerogel wurde bei 600°C in einem leichten Luftstrom mittels einem Röhrenofen 1 Stunde pyrolysiert. Das erhaltene, transparente Aerogel hatte eine Dichte von 0,18 g/cm³, eine spezifische Oberfläche nach BET von 450 m²/g und war hydrophil.

### Anwendungsbeispiele:

In den Anwendungsbeispielen werden hydrophile und hydrophobe Aerogele eingesetzt, wie sie gemäß den Herstellungsbeispielen 1 und 2 erhalten wurden.

### Beispiel 1:

### Benetzbarkeit von Aerogelen:

| Aerogel | Hydrophil | Hydrophob |
|---|---|---|
| Aceton | + | + |
| Ethanol | + | + |
| Ethylacetat | + | + |
| n-Hexan | + | + |
| Methanol | + | + |
| i-Propanol | + | + |
| Wasser | + | - |
| +: Benetzung; -: keine Benetzung | | |

### Beispiel 2:

### Wasseraufnahme von Aerogelen bei intensiver mechanischer Einarbeitung

| | Wasseraufnahme (%) | Beschreibung |
|---|---|---|
| Aerogel hydrophil | bis 240 | frei fließendes Pulver |
| | 280 | gelartige Konsistenz |
| | 300 | dünnflüssige Suspension |
| Aerogel hydrophob | bis 140 | frei fließendes Pulver |
| | 260 | dickflüssige Paste |
| | 320 | viskose weiße Suspension |

### Beispiel 3:

### Beladung von Aerogelen mit Na-Carboxyfluorescein:

5 g Aerogel werden mit 50 ml einer 1,5 %igen ethanolischen Na-Carboxyfluoresceinlösung versetzt und 2 Stunden lang gerührt. Nach Filtration wird der Rückstand bei Raumtemperatur unter Normaldruck getrocknet und das Produkt gesiebt. Man erhält ein frei fließendes Puiver.

| | Gehalt an Na-Carboxyfluorescein |
|---|---|
| Aerogel hydrophil | 6,2 % |
| Aerogel hydrophob | 5,7 % |

d.h. mindestens 38 % der angebotenen Stoffmenge wird aufgenommen.

### Beispiel 4:

### Freigabe von Na-Carboxyfluorescein aus Aerogelen:

Freigabeapparatur: Paddle (USP)

Medium: Wasser 37°C

| Freigabe | | | |
|---|---|---|---|
| Zeit (min) | 5 | 60 | 150 |
| Aerogel hydrophil | 51 % | 80 % | n.b. |
| Aerogel hydrophob | 13 % | 18 % | 38 % |

### Beispiel 5

### Beladung von Aerogelen mit pharmazeutischen Wirkstoffen

Beladung durch Suspendieren des Trägers (Aerogel hydrophil / hydrophob) in einer Wirkstoff-Lösung und anschließendes Trocknen (Normaldruck oder reduzierter Druck) oder Aufbringen einer Wirkstoff-Lösung auf den trockenen Träger und anschließendes Nachtrocknen. Man erhält ein freifließendes Pulver.
A) 1 g Aerogel vorlegen, 20 ml einer 5 %igen Furosemid Lösung (Aceton) unter Rühren zugeben, Lösungsmittel bei Normaldruck und bei Raumtemperatur abdampfen lassen
   Wirkstoffbeladung: 50 %
B) 1 g Aerogel vorlegen, 2 ml einer 5 %igen Furosemid Lösung (Aceton) unter Rühren zugeben, Lösungsmittel unter Normaldruck und bei Raumtemperatur abdampfen lassen, Wdh. bis zur gewünschten Beladung (z.B. viermal)
   Wirkstoffbeladung: 33,3 %
C) 1 g Aerogel vorlegen, Zugabe einer 5 %igen Furosemid Lösung (Aceton) bis ein eben noch rieselfähiges Pulver entsteht, Nachtrocknen (Normaldruck oder reduziertem Druck)
   Wirkstoffbeladung: 13,0 %
D) 1 g Aerogel vorlegen, 15 ml einer 1,3 %igen Furosemid-Natrium Lösung (Aceton) unter Rühren zugeben, Lösungsmittel bei Normaldruck und bei Raumtemperatur abdampfen lassen
   Wirkstoffbeladung: 16,6 %
E) 1 g Aerogel vorlagen, 15 ml einer 1,3 %igen Penbutulolhemisulfat Lösung (Methanol/Ethanol 1:1) unter Rühren zugeben, Lösungsmittel bei Normaldruck und bei Raumtemperatur abdampfen lassen
   Wirkstoffbeladung: 16,6 %
F) 1 g Aerogel vorlegen, 20 ml einer 1 %igen Hoe 277* Lösung (Ethanol) unter Rühren zugeben, Lösungsmittel bei Normaldruck und bei Raumtemperatur abdampfen lassen
   Wirkstoffbeladung: 16,6 %
G) 1 g Aerogel vorlegen, 13,5 ml einer 0,75 %igen Methylprednisolon Lösung (Ethanol) unter Rühren zugeben, Lösungsmittel bei Normaldruck und bei Raumtemperatur abdampfen lassen
   Wirkstoffbeladung: 9,1 %

* Pyridin-2,4-dicarbonsäure-N,N,-(3-methoxy-propyl)amid (beschrieben in der EP-A-0 409 119)

### Beispiel 6

Freisetzung von pharmazeutischen Wirkstoffen aus Aerogelen
A) Freisetzung von Methylprednisolon aus hydrophobem Aerogel

| | |
|---|---|
| Beladung | 9,1 % Methylprednisolon |
| Freigabemethode | Blattrührermethode DAB 10 |
| Medium | Salzsäure 0,1 N |

| Zeit (min) | Freisetzung Methylprednisolon | Freisetzung Methylprednisolon |
|---|---|---|
| | Reinsubstanz (%) | aus hydrophobem Aerogel (%) |
| 15 | 18,8 | 16,8 |
| 120 | 84,1 | 41,1 |
| 480 | 91,5 | 58,7 |
| 1440 | 92,3 | 77,2 |

B) Freisetzung von Methylprednisolon aus Aerogelen

| | |
|---|---|
| Beladung | 9,1 % Methylprednisolon |
| Freigabemethode | Blattrührermethode DAB 10 |
| Medium | Phosphatpuffer pH 7,5 |

| Zeit (min) | Freisetzung Methylprednisolon Reinsubstanz (%) | Freisetzung Methylprednisolon aus hydrophilem Aerogel % | Freisetzung Methylprednisolon aus hydrophobem Aerogel % |
|---|---|---|---|
| 3 | 3,9 | 56,5 | 1,6 |
| 6 | 12,5 | 68,2 | 3,1 |
| 15 | 33,2 | 75,3 | 6,5 |
| 30 | 53,9 | 78,6 | 11,6 |

C) Freisetzung von Hoe 277 aus Aerogelen

| | |
|---|---|
| Beladung | 16,6 % Hoe 277 |
| Freigabemethode | Blattrührermethode DAB 10 |
| Medium | Salzsäure 0,1 N |

| Zeit (min) | Freisetzung HOE 277 aus hydrophilem Aerogel (%) | Freisetzung HOE 277 aus hydrophobem Aerogel (%) |
|---|---|---|
| 6 | 94,3 | 20,8 |
| 15 | 94,3 | 24,9 |
| 30 | 94,8 | 28,9 |

D) Freisetzung von Furosemid aus Aerogelen

| | |
|---|---|
| Beladung | 50 % Furosemid |
| Freigabemethode | Blattrührermethode DAB 10 |
| Medium | Wasser |

| Zeit (min) | Freisetzung Furosemid Reinsubstanz (%) | Freisetzung Furosemid aus hydrophobem Aerogel (%) |
|---|---|---|
| 3 | 8,7 | 2,3 |
| 6 | 15,7 | 2,7 |
| 15 | 29,9 | 5,5 |
| 30 | 49,5 | 9,0 |

### Beispiel 7:

### Herstellung von Aerogel Tabletten:

| | | |
|---|---|---|
| Rezeptur | mikrokrist. Cellulose | 1 Teil |
| | Maisstärke | 1 Teil |
| | Mg-stearat | 0,01 Teile |
| | Aerogel* | 0,05 Teile |

| | | |
|---|---|---|
| *: Na-Carboxyfluorescein haltige Aerogele aus Bsp. 3 (hydrophil bzw. hydrophob) | | |

Verfahren: Mischen der Komponenten und anschließend Direkttablettierung mit einer Tabletten-Exzenterpresse zu runden, biptanaren Tabletten (Ø 6 mm) mit einer Masse von 100 mg und einer radialen Durckfestigkeit von 50 und 100 N.

Es lassen sich sowohl mit hydrophilen als auch mit hydrophoben Aerogelen problemlos Tabletten herstellen.

### Beispiel 8:

### Herstellung von Aerogel Kapseln:

| | | |
|---|---|---|
| Rezeptur | Aerogel* | 2 Teile |
| | Lactose 1 H₂O D 80 ** | 98 Teile |

| | | |
|---|---|---|
| *: Na-Carboxyfluorescein haltige Aerogele aus Bsp. 3 (hydrophil bzw. hydrophob) | | |
| **: Fa. Meggle, Wasserburg Verfahren: Handabfüllung | | |

Es werden sowohl mit hydrophilen als auch mit hydrophoben Aerogelen freifließende Pulver erhalten, die sich problemlos in Kapseln abfüllen lassen.

### Beispiel 9 (a, b, c und d):

### Herstellung von hydrophilen bzw. hydrophoben Aerogel Zäpfchen:

| | | |
|---|---|---|
| Rezeptur | Aerogel* | 2 Teile |
| | Witepsol** | 98 Teile |

| | | |
|---|---|---|
| *: Na-Carboxyfluorescein haltige Aerogele aus Bsp. 3 (hydrophil (a, b) bzw, hydrophob (c, d)) | | |
| **: Witepsol H 12 (a, c) bzw. Witepsol W 45 (b, d), Hüls AG, Witten Verfahren: Schmelzgießverfahren | | |

Die hydrophilen bzw. hydrophoben Aerogele lassen sich ohne Schwierigkeiten in die beiden Zäpfchengrundlagen einarbeiten.

### Beispiel 10 (a, b, c und d):

### Herstellung von wasserhaltigen Aerogel Zäpfchen:

| | | |
|---|---|---|
| Rezeptur | Aerogel* | 1 Teil |
| | Fluorescein-Natrium Lsg. 1,5 %ig | 1 Teil |
| | Witepsol** | 98 Teile |

| | | |
|---|---|---|
| *: Aerogele (hydrophil (a, b) bzw. hydrophob (c, d)) | | |
| **: Witepsol H 12 (a, c) bzw. Witepsol W 45 (b, d), Hüls AG, Witten Verfahren: Schmelzgießverfahren | | |

Die wäßrige Phase läßt sich ohne Schwierigkeiten in die beiden Zäpfchengrundlagen einarbeiten.

### Beispiel 11:

### Herstellung einer Aerogel Lotion:

| Rezeptur: | |
|---|---|
| Aerogel | 4,41 g |
| Propylenglykol | 8,82 g |
| Polysorbat 60 | 4,41 g |
| Polysorbat 65 | 4,41 g |
| Paraffinöl, dünnflüssig | 13,24 g |
| Polyacrylsäure | 0,22 g |
| Natronlauge 1 N | 0,88 g |
| Editinsäure, Tetranatrium Salz Dihydrat | 0,09 g |
| Methyl-4-hydroxybenzoat | 0,10 g |
| Propyl-4-hydroxybenzoat | 0,01 g |
| Wasser | 63,41 g |

Es entsteht, sowohl mit dem hydrophilen, als auch mit dem hydrophoben Aerogel eine weiße homogene Milch mit Peeling Effekt.

### Beispiel 12 (a und b):

### Herstellung von aerogelhaltigen Gelen

| | | |
|---|---|---|
| Rezeptur | Aerogel* | 11,0 g |
| | Miglyol 812 | 99,0 g |

| | | |
|---|---|---|
| *: Aerogele (hydrophil (a) bzw. hydrophob (b)) | | |

Es entstehen klare bzw. schwach opaleszente Gele mit Peeling-Effekt.

### Beispiel 13:

### Beladung von hydrophilem bzw. hydrophobem Aerogel mit lipophilen Stoffen

| | |
|---|---|
| Rezeptur | Aerogel 3 g |
| | Sudanrot 0,5 g |
| | Isopropanol 80 g |

Sudanrot wird in Isopropanol gelöst und mit dem entsprechenden Aerogel 2 Stunden lang gerührt. Nach Abtrennung der überschüssigen, flüssigen Phase wird das Aerogel bei Raumtemperatur und Normaldruck getrocknet. Man erhält ein freifließendes sudanrothaltiges Pulver.

### Beispiel 14:

### Dispergieren von lipophilen Stoffen in hydrophilen Medien

| | | |
|---|---|---|
| A) | Aerogel hydrophil mit Sudanrot | 1 Teil |
| | Wasser | 99 Teile |

Man erhält eine homogene rote Suspension. Eine Agglomeration von Partikeln wird nicht beobachtet.

| | | |
|---|---|---|
| B) | (Vergleichsbeispiel) | |
| | Sudanrot | 0,1 Teile |
| | Wasser | 99 Teile |

Es findet auch nach intensivem Schütteln keine Benetzung oder Dispergierung von Sudanrot in Wasser statt. Das Produkt agglomeriert stark.

| | | |
|---|---|---|
| C) | Aerogel, hydrophob mit Sudanrot | 1 Teil |
| | Wasser | 99 Teile |

Man erhält eine homogene Verteilung des sudanrothaltigen Aerogels an der Oberfläche des Wassers, ohne daß Agglomerate auftreten.

### Beispiel 15:

### Beladung von Aerogel mit hydrophilen Stoffen

| | | hydrophob | hydrophil |
|---|---|---|---|
| Rezeptur: | Aerogel | 1 Teil | 1 Teil |
| | Wasser | 1,4 Teile | 2 Teile |
| | Wassergehalt (%) | 58 | 66 |

Nach intensivem Verreiben erhält man ein homogenes freifließendes Pulver.

### Beispiel 16:

### Dispergieren von hydrophilen Stoffen in hydrophoben Medien

| | | |
|---|---|---|
| A) | Aerogel (wasserhaltig) (hydrophil bzw. hydrophob) | 1 Teil |
| | Sesamöl | 50 Teile |

Man erhält unter leichtem Rühren eine homogene, wasserhaltige Suspension. Wasserabscheidungen sind auch nach 24 Stunden nicht beobachtbar.

| | | |
|---|---|---|
| B) | Wasser | 0,1 Teile |
| | Sesamöl | 50 Teile |

Auch unter starkem Rühren ist keine homogene Verteilung des Wassers (hydrophiler Modellstoff) in Sesamöl möglich. Nach kurzer Zeit aggregieren dispergierte Wassertröpfchen. Es besteht immer eine deutliche Phasentrennung.

### Beispiel 17:

### Herstellung von hydrophilen Aerogel-Zäpfchen mit eingeschlossener hydrophiler Phase

| | | |
|---|---|---|
| Rezeptur | Aerogel hydrophil | 1 Teil |
| | Fluorescein Na Lsg. 1,5 %ig | 2 Teile |

Nach Verreiben erhält man ein freifließendes Pulver, das bis zu einem Anteil von 33 % (=̂ 22 % hydrophiler Phase) problemlos und homogen in aufgeschmolzene Zäpfchengrundmassen (Witepsol H 12 bzw. W 45) einarbeitbar ist. Es tritt keine hydrophile Phase aus den Zäpfchen aus. Witepsol H 12 Zäpfchen mit 5 % Natrium-Fluorescein-Lösung (1,5 %ig) sind hingegen inhomogen. Die hydrophile Phase tritt aus dem Zäpfchen aus.

## Patentansprüche

1. Verwendung von durch Silylierung oberflächenmodifizierten anorganischen Aerogelen als Hilfsstoff und/oder Trägermaterial fiir pharmazeutische Wirkstoffe und/oder Zubereitungen.

2. Verwendung von Aerogelen gemäß Anspruch 1 als pharmazeutischer Hilfsstoff für feste, halbfeste und/oder flüssige orale Zubereitungen.

3. Verwendung von Aerogelen gemäß Anspruch 1 als pharmazeutischer Hilfsstoff für topische Zubereitungen.

4. Verwendung von Aerogelen gemäß Anspruch 3 für Zubereitungen zur dermalen, vaginalen, rektalen und oromucosalen Applikation.

5. Verwendung von Aerogelen gemäß Anspruch 1 als pharmazeutisches Trägermaterial zur beschleunigten, kontrollierten und/oder retardierten Freisetzung von Arzneistoffen.

6. Verwendung von Aerogelen gemäß Anspruch 5 für magensaftflotierende Arzneiformen.

7. Verwendung von Aerogelen gemäß Anspruch 5 oder 6 zur Verarbeitung von flüssigen Arzneistoffen.

8. Verwendung von Aerogelen gemäß mindestens einem der vorhergehenden Ansprüche, **dadurch gekennzeichnet, dass** Aerogel-Partikel mit Porositäten über 60 % und Dichten unter 0,6 g/cm³ eingesetzt werden.

## Claims

1. The use of inorganic aerogels, surface-modified by silylation, as an auxiliary and/or excipient for pharmaceutical active ingredients and/or preparations.

2. The use of aerogels according to claim1 as a pharmaceutical auxiliary for solid, semi-solid, and/or liquid oral preparations.

3. The use of aerogels according to claim1 as a pharmaceutical auxiliary for topical preparations.

4. The use of aerogels according to claim 3 for preparations for dermal, vaginal, rectal and oromucosal administration.

5. The use of aerogels according to claim1 as a pharmaceutical excipient for the accelerated, controlled and/or delayed release of pharmaceuticals.

6. The use of aerogels according to claim 5 for pharmaceutical forms which float on gastric juice.

7. The use of aerogels according to claim 5 or 6 for the processing of liquid pharmaceuticals.

8. The use of aerogels according to at least one of the preceding claims, **characterised in that** aerogel particles having porosities of over 60% and densities of under 0.6 g/cm3 are employed.

## Revendications

1. Utilisation d'aérogels inorganiques modifiés en surface par silylation comme adjuvant et/ou matériau support pour des substances et/ou préparations pharmaceutiques.

2. Utilisation d'aérogels selon la revendication 1 comme adjuvant pharmaceutique pour des préparations orales solides, semi-solides et/ou liquides.

3. Utilisation d'aérogels selon la revendication 1 comme adjuvant pharmaceutique pour des préparations topiques.

4. Utilisation d'aérogels selon la revendication 3 pour l'administration dermique, vaginale, rectale et oromuqueuse.

5. Utilisation d'aérogels selon la revendication 1 comme matériau support pharmaceutique pour la libération accélérée, contrôlée et/ou retardée de substances médicamenteuses.

6. Utilisation d'aérogels selon la revendication 5 pour des formes médicamenteuses flottant dans le suc gastrique.

7. Utilisation d'aérogels selon la revendication 5 ou 6 pour la réalisation de substances médicamenteuses liquides.

8. Utilisation d'aérogels selon au moins une des revendications précédentes, **caractérisée en ce qu'**on utilise des particules d'aérogel avec des porosités supérieures à 60 % et des masses volumiques inférieures à 0,6 g/cm³.
